(19) Europäisches Patentamt
European Patent Office
Office européen des brevets

(11) Numéro de publication : **0 384 818 B1**

# (12) FASCICULE DE BREVET EUROPEEN

(45) Date de publication du fascicule du brevet : **01.09.93 Bulletin 93/35**

(51) Int. Cl.$^5$ : **C07C 15/16,** C07C 2/86, H01B 3/22

(21) Numéro de dépôt : **90400442.1**

(22) Date de dépôt : **19.02.90**

## (54) Compositions à base de polyphénylméthanes, leur procédé de fabrication et leur application comme diélectrique.

(30) Priorité : **20.02.89 FR 8902166**

(43) Date de publication de la demande : **29.08.90 Bulletin 90/35**

(45) Mention de la délivrance du brevet : **01.09.93 Bulletin 93/35**

(84) Etats contractants désignés : **AT BE CH DE DK ES FR GB GR IT LI LU NL SE**

(56) Documents cités :
**EP-A- 0 136 230**
**EP-A- 0 299 866**
**EP-A- 0 299 867**

(73) Titulaire : **ELF ATOCHEM S.A.**
**4 & 8, Cours Michelet La Défense 10**
**F-92800 Puteaux (FR)**

(72) Inventeur : **Berger, Noelle**
**Frênes 4, Charrière Blanche**
**F-69130 Ecully (FR)**
Inventeur : **Commandeur, Raymond**
**Le Rocher, Avenue de Vénaria**
**F-38220 Vizille (FR)**
Inventeur : **Jay, Pierre**
**89, Rocade des Monts d'Or**
**F-69370 Saint-Didier Au Mont D'Or (FR)**

**EP 0 384 818 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne des compositions à base de polyphénylméthanes, leur procédé de fabrication et leur application comme diélectrique. Elle concerne plus particulièrement des mélanges de mono et dibenzyltoluène, mono et dibenzylbenzène et pouvant contenir du triphénylméthane, du ditolylphénylméthane, du tolyldiphénylméthane ou leurs homologues supérieurs.

La demande de brevet EP 259798 décrit des mélanges de monobenzyltoluène d'éthylbiphényl et/ou de 1-1 diphényléthane avec d'autres biphényls ou d'autres diphénylméthanes ayant au plus 17 atomes de carbone.

La demande de brevet EP 262456 décrit des mélanges de monobenzyltoluène et d'un diphénylméthane substitué par des alkyls, la structure complète ayant entre 15 et 17 atomes de carbone.

La demande de brevet EP 172537 décrit des mélanges d'isomères du benzylbenzène.

La demande de brevet EP 282083 décrit des mélanges de monobenzyltoluène et de ditolylméthane.

Toutes ces compositions décrites sont obtenues par des réactions d'addition ou de couplage de réactifs purs nécessitant ensuite des séparations difficiles.

La demande de brevet EP 136230 décrit des mélanges de monobenzyltoluène, dibenzyltoluène et ditolylphénylméthane utilisables comme fluides diélectriques. Elle décrit aussi un procédé pour obtenir très simplement ces mélanges.

On a maintenant trouvé de nouvelles compositions à base de polyphénylméthanes qui possèdent des propriétés diélectriques meilleures que l'art antérieur. Un autre but de l'invention est de fournir des compositions utilisables à basse température.

Un autre but de l'invention est aussi de fournir des compositions très faciles à préparer.

L'invention est donc une composition à base de deux oligomères $A_1$ et $A_2$ tels que :

- $A_1$ est un isomère ou un mélange d'isomères de formule :

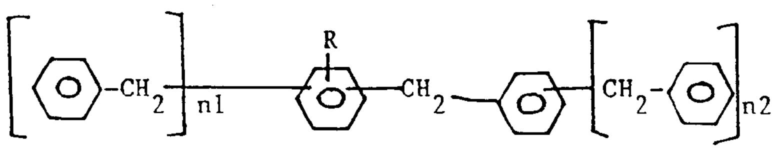

avec $n_1$ et $n_2$ = 0, 1 et 2 sachant que $n_1 + n_2$ est inférieur ou égal à 3 et R représente un hydrogène.

- $A_2$ est un isomère ou un mélange d'isomères de même formule générale que $A_1$ sauf que R représente un méthyle et $n_1$ et $n_2$ sont remplacés par $q_1$ et $q_2$ et ont la même signification, caractérisée en ce que l'un au moins des oligomères $A_1$ et $A_2$ comprend un isomère ayant trois noyaux benzéniques.

L'oligomère $A_2$ peut être par exemple du métabenzyltoluène ou un mélange de deux isomères du benzyltoluène ou un mélange des trois isomères du benzyltoluène. Ce peut être aussi un isomère bien précis avec $q_1 = 1$ et $q_2 = 0$, tel que le 3,5-dibenzyltoluène ou un mélange de tous les isomères tels que $q_1 = 1$ et $q_2 = 0$ ou un mélange des isomères tels que $q_1 = 0$ et $q_2 = 1$.

$A_2$ peut aussi être un mélange d'isomères dans lesquels $q_1$ et $q_2$ ont plusieurs significations, par exemple un mélange constitué de 64 % en poids de dibenzyltoluène, de 22 % en poids du mélange d'isomères tels que $q_1 + q_2 = 1$, de 10% en poids du mélange d'isomères tels que $q_1 + q_2 = 2$ et de 4 % en poids du mélange d'isomères tels que $q_1 + q_2 = 3$. Ce peut être aussi toute combinaison des isomères ou mélanges d'isomères décrits plus haut.

Il en est de même pour l'oligomère $A_1$ qui est donc un isomère ou un mélange d'isomères du benzylbenzène ou de ses homologues supérieurs. La composition selon l'invention est telle que l'un au moins des oligomères $A_1$ ou $A_2$ est un isomère qui a au moins 3 noyaux benzéniques dans sa structure ou un mélange d'isomères ayant 3 noyaux benzéniques, ou un mélange comprenant au moins un isomère ou des isomères ayant 3 noyaux benzéniques.

Le produit de l'invention peut être un mélange d'un oligomère $A_1$ quelconque et l'oligomère $A_2$ étant soit du dibenzyltoluène, c'est-à-dire un ou des isomères dans lesquels $q_1 = 1$ et $q_2 = 0$, soit du tolyl(benzylphényl)méthane, c'est-à-dire un ou des isomères dans lesquels $q_1 = 0$ et $q_2 = 1$.

Avantageusement, le produit de l'invention est une composition comprenant une des combinaisons suivantes des oligomères $A_1$ et $A_2$ :

a) $A_1$ tel que $n_1 = n_2 = 0$
$A_2$ tel que $q_1 + q_2 = 1$

b) $A_1$ tel que $n_1 + n_2 = 1$
$A_2$ tel que $q_1 = q_2 = 0$

c) $A_1$ tel que $n_1 + n_2 = 1$

$A_2$ tel que $q_1 + q_2 = 1$

Bien que les proportions de $A_1$ et $A_2$ puissent être quelconques, avantageusement la quantité de $A_2$ est supérieure à la quantité de $A_1$ et de préférence, comprise entre 60 et 80 % en poids de la quantité totale de $A_1$ et $A_2$.

L'invention concerne aussi une composition à base des deux oligomères $A_1$ et $A_2$, caractérisée en ce qu'elle comprend en plus au moins un oligomère choisi parmi les oligomères $B_1$, $B_2$ et $B_3$ suivants :

- $B_1$ est un isomère ou un mélange d'isomères de formule :

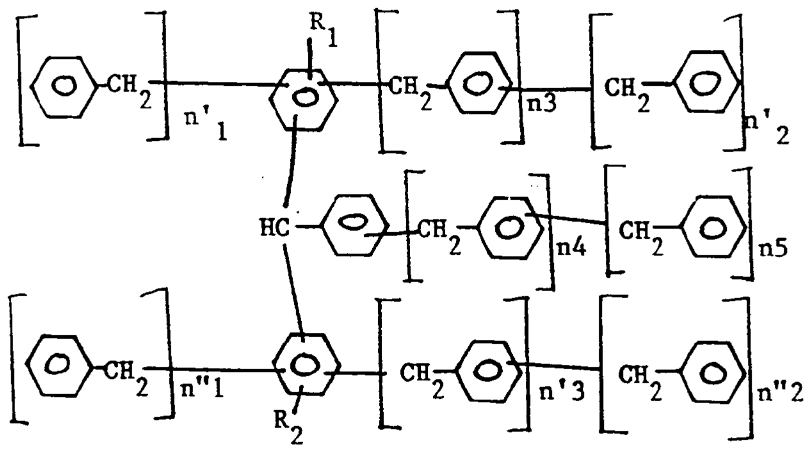

avec :

$n'_1$, $n''_1$ et $n_4$ = 0, 1 et 2

$n'_2$, $n''_2$, $n_3$, $n'_3$ et $n_5$ = 0 et 1

sachant que $n'_1 + n''_1 + n'_2 + n''_2 + n_3 + n'_3 + n_4 + n_5$ est inférieur ou égal à 2.

$R_1$ et $R_2$ représentent un hydrogène.

$B_2$ est un isomère ou un mélange d'isomères de même formule générale que $B_1$ sauf que $R_1$ et $R_2$ représentent un méthyle et les coefficients n sont remplacés par q et ont la même signification.

$B_3$ est un isomère ou un mélange d'isomères de même formule générale que $B_1$ sauf que $R_1$ et $R_2$ sont différents et représentent un hydrogène ou un méthyle et les coefficients n sont remplacés par r et ont la même signification.

L'oligomère $B_1$ peut être dans sa forme la plus simple du triphénylméthane. Ce peut être aussi un isomère bien précis du (benzylphényl)diphénylméthane, c'est-à-dire $n'_1 = 1$ et tous les autres coefficients valant zéro ou un mélange de plusieurs isomères de ce produit. $B_1$ peut aussi être un mélange d'isomères dans lesquels les coefficients n ont plusieurs significations, par exemple, tel que 72 % en poids de $B_1$ est constitué d'isomères dont la somme :

$n'_1 + n''_1 + n'_3 + n_3 + n'_2 + n''_2 + n_4 + n_5$ vaut 0

21 % en poids est constitué d'isomères dont la somme précédente vaut 1 et le reste est constitué d'isomères dont la somme précédente vaut 2. $B_1$ peut aussi être un isomère ou un mélange d'isomères représenté par un jeu bien précis des coefficients n. Ce peut être aussi un mélange qui est une combinaison de deux ou plusieurs mélanges cités ci-dessus. Les oligomères $B_2$ et $B_3$ se définissent de la même façon.

Des compositions particulièrement avantageuses sont formées de $A_1$, $A_2$ et $B_2$. Parmi ces compositions, on préfère les compositions suivantes :

a) $A_1$ tel que $n_1 = n_2 = 0$

$A_2$ tel que 60 à 90 % en poids de $A_2$ soit constitué d'isomères avec $n_1 + n_2 = 0$

$B_2$ quelconque

b) $A_1$ tel que plus de 50 % en poids de $A_1$ soit constitué d'isomères avec $n_1 + n_2 = 0$

$A_2$ et $B_2$ comme dans a)

Parmi les compositions à base de $A_1$, $A_2$ et de l'un au moins des oligomères $B_1$, $B_2$ et $B_3$, celle comprenant les 5 oligomères $A_1$, $A_2$, $B_1$, $B_2$ et $B_3$ est particulièrement avantageuse et de façon inattendue est très facile à fabriquer.

La présente invention concerne aussi la composition formée par le mélange $A_1$, $A_2$, $B_1$, $B_2$.

Toutes les compositions qu'on vient de décrire peuvent être utilisées comme fluide diélectrique, en particulier dans les condensateurs. Toutes ces compositions peuvent être purifiées, par exemple par mise en

contact avec une terre décolorante, une bentonite ou un produit équivalent jusqu'à l'obtention d'une résistivité suffisament élevée et en général être purifiées selon les techniques habituelles de purification des liquides à usage diélectrique. On peut aussi ajouter aux compositions de l'invention les additifs habituels des liquides à usage diélectriques tels que des époxydes, des antioxydants, du type ditertiobutyl paracresol, ou les dérivés de l'anthraquinone. La présente invention concerne aussi des procédés de synthèse de toutes ces compositions.

Les oligomères $A_1$, $A_2$ peuvent être préparés par réaction du chlorure de benzyle $C_6H_5CH_2Cl$ sur le toluène pour $A_2$ et sur le benzène pour $A_1$. Quant aux oligomères $B_1$, $B_2$ et $B_3$ ils peuvent être préparés par réaction du chlorure de benzylidène $C_6H_5CHCl_2$ sur le benzène, le toluène, le chlorure de benzyle et les oligomères $A_1$ et $A_2$.

Le chlorure de benzyle et le chlorure de benzylidène sont des produits connus. Par exemple, on peut effectuer la chloration radicalaire du toluène suivie d'une distillation pour séparer $C_6H_5CH_2Cl$ et $C_6H_5CHCl_2$.

Puis, quand on a obtenu les différents oligomères A et B, il suffit de les mélanger pour obtenir les compositions de l'invention.

Un procédé particulièrement commode pour obtenir le mélange des oligomères $A_2$ et $B_2$ est décrit dans le brevet européen EP 136230 et consiste, dans une première étape, à faire réagir le chlore sur le toluène par réaction radicalaire en présence de générateur de radicaux libres et dans une seconde étape, à soumettre le produit de réaction de cette première étape à l'action d'un halogénure minéral ou d'un acide minéral.

L'invention concerne aussi un procédé de préparation du mélange d'oligomères $A_1 + A_2$ caractérisé en ce qu'on met en contact du chlorure de benzyle avec du benzène et du toluène en présence d'un halogénure minéral ou d'un acide minéral.

Cette réaction a lieu en pratique à une température comprise entre 30 et 110°C, et de préférence entre 50 et 100°C. Parmi les halogénures minéraux, on peut utiliser le chlorure ferrique, le trichlorure d'antimoine, le tétrachlorure de titane ou encore le chlorure d'aluminium à des teneurs pondérales par rapport au milieu réactionnel compris habituellement entre 50 ppm et 1 % et de préférence entre 100 ppm et 0,5 %. Les acides minéraux peuvent également être utilisés : l'acide sulfurique par exemple à une concentration pondérable comprise entre 70 et 95 %. Il est aussi possible d'employer des zéolithes ou encore certains oxydes minéraux.

On peut aussi préparer $A_1 + A_2$ selon une variante du procéde précédent, caractérisé en ce que :

a) on fait réagir le chlore sur du toluène et du benzène en présence d'un générateur de radicaux libres,

b) on élimine le chlorure de benzylidène,

c) on soumet le produit obtenu à l'action d'un halogénure minéral ou d'un acide minéral.

La mise en oeuvre est semblable aux préparations précédentes. Pour l'étape b) il est avantageux de procéder par distillation, par exemple en recueillant la fraction légère du mélange obtenu en a). Cette fraction légère est constituée de benzène, de toluène et de chlorure de benzyle, il suffit alors d'y ajouter (i) un halogénure minéral ou un acide minéral ou (ii) un produit contenant cet halogénure ou cet acide.

On ne sortirait pas du cadre de l'invention en effectuant l'étape a) sans benzène et en le rajoutant à l'étape c). On peut aussi à l'étape c) rajouter du toluène.

L'invention concerne aussi un procédé de préparation du mélange des oligomères $A_1$, $A_2$, $B_1$, $B_2$ et $B_3$, caractérisé en ce que :

a) on fait réagir le chlore sur un mélange de benzène et de toluène par réaction radicalaire en présente d'un générateur de radicaux libres,

b) puis on soumet le produit de réaction précédent à l'action d'un halogénure minéral ou d'un acide minéral.

Comme précédemment, pourvu que le mélange benzène-toluène contienne au moins 15 % en moles de toluène, il n'y a pas chloration du benzène. La mise en oeuvre est semblable à celle des procédés précédents.

Les exemples suivants illustrent l'invention.

## EXEMPLE 1

Les essais consistent à soumettre des modèles de condensateurs à des vieillissements accélérés à tension et température élevées.

La sanction principale des essais est le nombre de condensateurs claqués. Dans certains tests, la durée de vieillissement est courte et la mesure de la rigidité des condensateurs non claqués en cours d'essai apporte une information supplémentaire sur la détérioration de l'isolation.

### I. ESSAI SUR CONDENSATEURS MIXTES

Pour cet essai on a fabriqué deux séries de dix condensateurs comportant deux couches de film de polypropylène lisse d'épaisseur 12 μm et une couche de papier de densité 1.0 et d'épaisseur 12 μm.

Ces deux séries de condensateurs ont été imprégnées, l'une avec un oligomère $A_2$ tel que 75 % en poids est du benzyloluène ($q_1 = q_2 = 0$) et 25 % en poids les isomères tels que $q_1 + q_2 = 1$ et contenant de l'éther/diglycidique du bisphénol A en proportion de 1 partie pour 100 parties de $A_2$, l'autre avec un mélange de $A_1$ et $A_2$ tel que :

a) $A_2$ représente 70 % en poids de $A_1 + A_2$,

b) $A_1$ est formé de 70 % en poids d'isomères tels que $n_1 = n_2 = 0$ et 30 % en poids d'isomères tels que $n_1 + n_2 = 1$.

1.1. Durée de vie

Aprés imprégnation et formation thermique les condensateurs ont été soumis à un vieillissement à 85°C sous 2700 V (75 V/μm) pendant 535 h. Après ce premier vieillissement aucun claquage n'ayant été observé l'essai a été poursuivi sous 3000 V (83,8 V/μm) afin d'en accroître la sévérité.

A ce jour, soit après 4400 h de vieillissement, on a obtenu les résultats suivants :

| Durée sous tension | Nombre de condensateurs en vie | |
|---|---|---|
| | $A_2$ | $A_1 + A_2$ |
| 0 | 10 | 10 |
| 695 | 9 | 10 |
| 1056 | 8 | 10 |
| 1458 | 7 | 10 |
| 3290 | 5 | 10 |
| 3450 | 5 | 9 |
| 3915 | 5 | 8 |
| 3990 | 5 | 7 |
| 4400 | 5 | 7 |

Ces résultats sont en faveur du mélange $A_1 + A_2$.

1.2. Evolutions de tan∂

La tan∂ des condensateurs a été mesurée après 535, 1460 et 4400 heures de vieillissement.

On a obtenu les résultats suivants :

| Durée de vieillissement | Tension de mesure (Volts) | Tan∂x$10^{-4}$ (85°C) $A_2$ | $A_1 + A_2$ |
|---|---|---|---|
| 535 | 5000 | 5,9 | 5,7 |
| | 1000 | 6,1 | 5,6 |
| | 2000 | 6,4 | 5,9 |
| | 3000 | 7,9 | 7,0 |
| 1460 | 500 | 9,0 | 6,4 |
| | 1000 | 9,4 | 6,5 |
| | 2000 | 10,3 | 6,8 |
| | 3000 | 12,5 | 8,1 |
| 4400 | 500 | 4,6 | 4,1 |
| | 1000 | 4,6 | 4,2 |
| | 2000 | 4,9 | 4,4 |
| | 3000 | 5,9 | 4,9 |

La tan∂ des condensateurs imprégnés au mélange $A_1 + A_2$ est significativement plus faible que celle des condensateurs imprégnés avec $A_2$ montrant que la détérioration est moins importante.

II. ESSAIS SUR CONDENSATEURS TOUT-FILM

Pour cet essai on a fabriqué trois séries de dix condensateurs comportant deux couches de film polypropylène rugueux d'épaisseur pondérale 13,5 µm.

Deux séries ont été imprégnées avec l'oligomère $A_2$ précédent, une série a été imprégnée avec le mélange $A_1 + A_2$ précédent.

2.1. Durée de vie

Après imprégnation et formation thermiques, ces condensateurs ont été soumis à un vieillissement à 90°C sous 2400 V (88,9 V/µm) pendant 500 heures.

On a obtenu les résultats suivants :

| Durée sous tension | Nombre de condensateurs en vie | |
|---|---|---|
| | $A_2$ | $A_1 + A_2$ |
| 0 | 20 | 10 |
| 160 | 19 | 10 |
| 188 | 18 | 10 |
| 259 | 17 | 10 |
| 418 | 17 | 9 |
| 479 | 16 | 9 |
| 500 | 16 | 9 |

Ces résultats montrent une légère différence en faveur du mélange $A_1 + A_2$.

2.2. Evolution de la tension de claquage

Pour confirmer ces résultats on a mesuré la tension de claquage des condensateurs encore en vie à la fin de l'essai de vieillissement.

La même mesure effectuée sur des condensateurs neufs donne une valeur de 11,5 kV. Toute détérioration de l'isolation au cours du vieillissement se traduit par une baisse de rigidité.

On a obtenu les résultats suivants :

- condensateurs imprégnés avec $A_2$ : 6,6 kV
- condensateurs imprégnés avec $A_1 + A_2$ : 9,4 kV

Ces résultats montrent de façon significative que la détérioration des condensateurs imprégnés avec $A_1 + A_2$ est bien plus faible.

EXEMPLE 2 - Synthèse d'un oligomère $A_1$

Dans un réacteur de 6 litres muni d'une agitation rotative, d'un réfrigérant ascendant d'un injecteur d'azote et d'une ampoule de coulage, on charge 54 moles de benzène (4212 g) et 1 g de $FeCl_3$. La masse dans le réacteur, mise sous couverture d'azote, est portée à 65°C. On introduit par l'intermédiaire de l'ampoule de coulage 6 moles de chlorure de benzyle (759 g) en 4 h. Le milieu réactionnel est maintenu encore 1 h sous agitation et on ajoute 0,25 g de $FeCl_3$. Le milieu réactionnel est maintenu encore 2 h à 70°C sous balayage d'azote. La quantité d'acide chlorhydrique dégagé est de 5,95 moles. Le benzène non réagi est ensuite éliminé par une distillation simple. Le polyphénylméthane brut (873 g) est ensuite traité par 1 % de $Na_2CO_3$ anhydre pendant 3 h à 275°C avec agitation. Le produit après traitement est soumis à une distillation avec une colonne de 30 cm de haut remplie par un garnissage constitué par des ressorts de verre sous un vide de 1 mm de mercure.

La fraction de composés à deux cycles aromatiques passe vers 85-95°C.

La fraction de composés à trois cycles aromatiques passe vers 170-200°C.

La fraction de composés à quatre cycles aromatiques passe vers 260-280°C (après avoir enlevé la colonne à distiller).

Le résidu représente 5,8 % du produit engagé. Les fractions de distillation sont mélangées et représentent 94 % du produit engagé. On obtient un oligomère $A_1$ dont la composition pondérale est la suivante :

| | |
|---|---|
| $n_1$ et $n_2 = 0$ | 66,5 % |
| $n_1 = 1$ $n_2 = 0$ | 27 % |
| $n_1$ et $n_2 = 1$ | 6,5 % |

Les caractéristiques sont les suivantes :

| | |
|---|---|
| Point de cristallisation | = + 3°C |
| Viscosité à 40°C | = 3,4 cps |
| Viscosité à 20°C | = 5,8 cps |
| Densité à 40°C | = 1,001 |
| Densité à 20°C | = 1,012 |

EXEMPLE 3 : Synthèse du mélange $A_1$ + $B_1$

Dans un appareillage identique à celui de l'exemple 2, on place dans l'ampoule de coulage 9,61 moles de chlorure de benzyle (1266 g) et 0,306 mole de chlorure de benzylidène (1266 g) qui est obtenu par photochloration de toluène (rapport molaire toluène/chlore = 4) et séparation du toluène non réagi par distillation.

Ce produit est mis en réaction dans les mêmes conditions que dans l'exemple 2 avec 50 moles de benzène (3900 g) et 1 g de $FeCl_3$. L'acide chlorhydrique recueilli est de 9,99 moles. Le produit obtenu après distillation du benzène non réagi représente 1304 g. Il est traité comme dans l'exemple 2 par le carbonate de sodium et soumis ensuite à la même distillation. Le résidu de distillation représente 14,2 % du produit engagé. Les fractions sont mélangées et représentent 85 % du produit engagé. Le produit ainsi obtenu est du type mélange d'oligomères $A_1$ et $B_1$.

Il est constitué pondéralement par :

- 53 % :
  . d'oligomères $A_1$ $n_1$ et $n_2 = 0$
- 31 % :
  . d'oligomères $A_1$ $n_1 = 1$ $n_2 = 0$
  . oligomères $B_1$ $n'_1 = n_2 = n''_1 = n''_2 = n_3 = n'_3 = n_4 = n_5 = 0$
- 16 % :
  . d'oligomères $A_1$ $n_1 = n_2 = 1$
  . oligomères $B_1$ $n'_1 + n'_2 + n''_1 + n''_2 + n_3 + n'_3 + n_4 + n_5 = 1$

Les caractéristiques sont les suivantes :

| | |
|---|---|
| Point de cristallisation | : -15°C |
| Viscosité à 20°C | : 8,1 cps |
| Densité à 20°C | : 1,019 |

EXEMPLE 4 : Préparation de $A_1$ + $A_2$ + $B_1$ + $B_2$ + $B_3$

Dans un réacteur de 1 l muni d'une agitation, d'un condenseur, d'un tube d'alimentation de chlore et d'une lampe Philips TLADK de 30 Watts disposé extérieurement, on place 5 moles de benzène (390 g) et 5 moles de toluène (460 g) ; on introduit ensuite 1,25 mole de chlore (78,1 g) en maintenant la température à 85°C durant 1 h. La quantité d'acide chlorhydrique dégagé est de 1,15 mole. L'analyse chromatographique montre que le benzène est parfaitement inerte pendant la chloration. Le mélange contient 16 % de chorure de benzyle et 0,52 % de chlorure benzylidène en poids.

Le milieu réactionnel est placé dans une ampoule de coulage et il est introduit en 1 h dans un réacteur de 1 l muni d'une agitation rotative contenant 0,166 mole de toluène (15,3 g), 0,166 mole de benzène (12,95 g) et 0,15 g de $FeCl_3$ à la température de 80°C. L'ensemble est maintenu encore 1 h à 80°C avec balayage d'azote. La quantité d'acide chlorhydrique dégagé est de 1,1 mole.

Le produit obtenu après distillation du benzène et du toluène non réagi représente 180 g. Il est traité comme dans l'exemple 1 par le carbonate de sodium et il est soumis à la même distillation. Le résidu de distillation représente 2,76 % du produit engagé. Les fractions de distillation sont mélangées et représentent 97,1 % du produit engagé. Le produit ainsi obtenu est du type mélange d'oligomères $A_1$, $A_2$, $B_1$, $B_2$ et $B_3$.

- 71,1 % d'oligomère $A_1$ + $A_2$ $n_1$ et $n_2 = 0$
  $q_1$ et $q_2 = 0$
- 24,3 % :
  . d'oligomère $A_1$ + $A_2$ $n_1 = 1$ et $n_2 = 0$
  $q_1 = 1$ et $q_2 = 0$
  . oligomères $B_1$ + $B_2$ + $B_3$ tels que tous les coefficients n, p et r sont nuls
- 4,6 %
  . d'oligomères $A_1$ + $A_2$ $n_1 = n_2 = 1$
  $q_1 = q_2 = 1$
  . d'oligomères $B_1$ + $B_2$ + $B_3$ tels que la somme de tous les coefficients n est égale à 1, la somme de tous les coefficients q est égale à 1, la somme de tous les coefficients r est égale à 1.

Dans l'oligomère $A_1$ + $A_2$, la répartition pondérale entre $A_2$ est de 85/15 (déterminée par chromatographie en phase gazeuse) pour la valeur $n_1$ et $n_2 = 0$.

Les caractéristiques sont les suivantes :

Cristallisation < à - 32°C
Viscosité à 20°C = 6,7 cps
Viscosité à 20°C = 0,998

EXEMPLE 5

Nous avons effectué quelques mélanges du produit $A_1$ de l'exemple 2 avec un produit $A_2 + B_2$ préparé selon la technique décrite dans le brevet EP 136 230 et dont la composition en poids par rapport à la formule générale est la suivante :

- 75 % d'oligomère $A_2$ $q_1$ et $q_2 = 0$
- 23 % :
  - . d'oligomère $A_2$ $q_1 = 1$ et $n_2 = 0$
  - . oligomères $B_2$ $q'_1=q'_2=q''_1=q''_2=q_3=q'_3=q_4=q_5= 0$
- 2 %
  - . d'oligomères $A_2$ $q_1 = q_2 = 1$
  - . oligomères $B_2$ $q'_1+q'_2+q''_1+q''_2+q_3+q'_3+q_4+q_5= 1$

La teneur en oligomères $B_2$ :

$$q'_1 = q'_2 = q''_1 = q''_2 = q_3 = q'_3 = q_4 = q_5 = 0$$

est de 2,5 % déterminée par analyse chromatographique.

Le point de cristallisation est inférieur à - 40°C. La viscosité à 20°C est de 6,5 cps et la densité de 1,006.

| | Composition Pondérale | Début de cristallisation |
|---|---|---|
| Produit $A_1$ | 100 | + 3°C |
| Produit $A_1$<br>Produit $A_2 + B_2$ | 90<br>10 | - 5°C |
| Produit $A_1$<br>Produit $A_2 + B_2$ | 80<br>20 | - 10°C |
| Produit $A_1$<br>Produit $A_2 + B_2$ | 50<br>50 | - 30°C |

**Revendications**

**1.** Composition à base de deux oligomères $A_1$ et $A_2$ tels que :

- $A_1$ est un isomère ou un mélange d'isomères de formule :

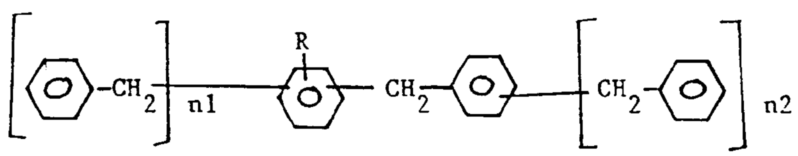

avec $n_1$ et $n_2$ = 0, 1 et 2 sachant que $n_1 + n_2$ est inférieur ou égal à 3 et R représente un hydrogène.

- $A_2$ est un isomère ou un mélange d'isomères de même formule générale que $A_1$ sauf que R représente un méthyle et $n_1$ et $n_2$ sont remplacés par $q_1$ et $q_2$ et ont la même signification, caractérisée en ce que l'un au moins des oligomères $A_1$ et $A_2$ comprend un isomère ayant trois noyaux benzéniques.

2. Composition selon la revendication 1, caractérisée en ce que l'oligomère $A_1$ est tel que $n_1 = n_2 = 0$ et l'oligomère $A_2$ est tel que $q_1 + q_2 = 1$.

3. Composition selon la revendication 1, caractérisée en ce que l'oligomère $A_1$ est tel que $n_1 + n_2 = 1$ et l'oligomère $A_2$ est tel que $q_1 = q_2 = 0$.

4. Composition selon la revendication 1, caractérisée en ce que l'oligomère $A_1$ est tel que $n_1 + n_2 = 1$ et l'oligomère $A_2$ est tel que $q_1 + q_2 = 1$.

5. Une composition à base des deux oligomères $A_1$ et $A_2$ tel que
   - $A_1$ est un isomère ou un mélange d'isomères de formule :

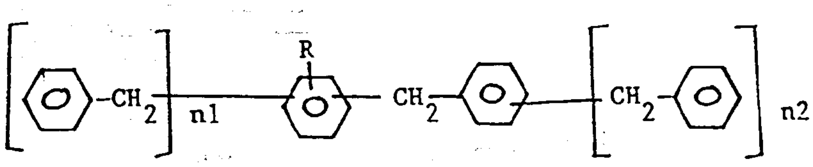

avec $n_1$ et $n_2$ = 0, 1 et 2 sachant que $n_1 + n_2$ est inférieur ou égal à 3 et R représente un hydrogène.
   - $A_2$ est un isomère ou un mélange d'isomères de même formule générale que $A_1$ sauf que R représente un méthyle et $n_1$ et $n_2$ sont remplacés par $q_1$ et $q_2$ et ont la même signification,,

caractérisée en ce qu'elle comprend en plus au moins un oligomère choisi parmi les oligomères $B_1$, $B_2$ et $B_3$ suivants :
   - $B_1$ est un isomère ou un mélange d'isomères de formule :

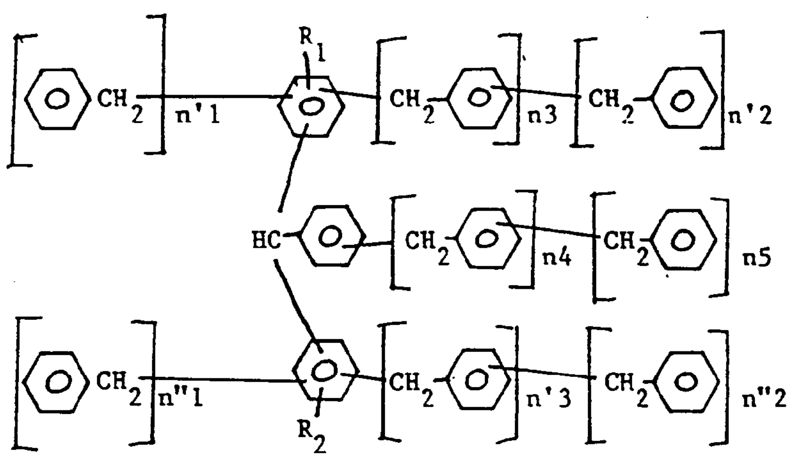

avec :

$n'_1$, $n''_1$ et $n_4$ = 0, 1 et 2

$n'_2$, $n''_2$, $n_3$, $n'_3$ et $n_5$ = 0 et 1

sachant que $n'_1 + n''_1 + n'_2 + n''_2 + n_3 + n'_3 + n_4 + n_5$ est inférieur ou égal à 2.

$R_1$ et $R_2$ représentent un hydrogène.

$B_2$ est un isomère ou un mélange d'isomères de même formule générale que $B_1$ sauf que $R_1$ et $R_2$ représentent un méthyle et les coefficients n sont remplacés par q et ont la même signification.

$B_3$ est un isomère ou un mélange d'isomères de même formule générale que $B_1$ sauf que $R_1$ et $R_2$ sont différents et représentent un hydrogène ou un méthyle et les coefficients n sont remplacés par r et ont la même signification

6. Composition selon la revendication 5, caractérisée en ce qu'elle comprend les oligomères $A_1$, $A_2$ et $B_2$.

7. Composition selon la revendication 5, caractérisée en ce qu'elle comprend les oligomères $A_1$, $A_2$, $B_1$, $B_2$ et $B_3$.

8. Application des compositions selon l'une des revendications 1 à 7 comme fluide diélectrique.

**9.** Procédé de préparation du mélange d'oligomères $A_1$ + $A_2$ tel que

- $A_1$ est un isomère ou un mélange d'isomères de formule :

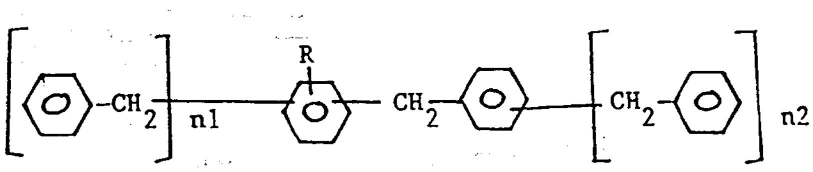

avec $n_1$ et $n_2$ = 0, 1 et 2 sachant que $n_1 + n_2$ est inférieur ou égal à 3 et R représente un hydrogène.

- $A_2$ est un isomère ou un mélange d'isomères de même formule générale que $A_1$ sauf que R représente un méthyle et $n_1$ et $n_2$ sont remplacés par $q_1$ et $q_2$ et ont la même signification,,

caractérisé en ce qu'on met en contact du chlorure de benzyle avec du benzène et du toluène en présence d'un halogénure minéral ou d'un acide minéral.

**10.** Procédé de préparation d'un mélange $A_1$ + $A_2$ tel que

- $A_1$ est un isomère ou un mélange d'isomères de formule :

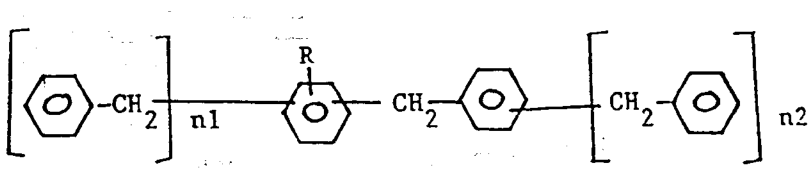

avec $n_1$ et $n_2$ = 0, 1 et 2 sachant que $n_1 + n_2$ est inférieur ou égal à 3 et R représente un hydrogène.

- $A_2$ est un isomère ou un mélange d'isomères de même formule générale que $A_1$ sauf que R représente un méthyle et $n_1$ et $n_2$ sont remplacés par $q_1$ et $q_2$ et ont la même signification,,

caractérisé en ce que :

a) on fait réagir le chlore sur du toluène et du benzène en présence d'un générateur de radicaux libres,
b) on élimine le chlorure de benzylidène,
c) on soumet le produit obtenu à l'action d'un halogénure minéral ou d'un acide minéral.

**11.** Procédé de préparation d'un mélange $A_1$ + $A_2$ tel que

- $A_1$ est un isomère ou un mélange d'isomères de formule :

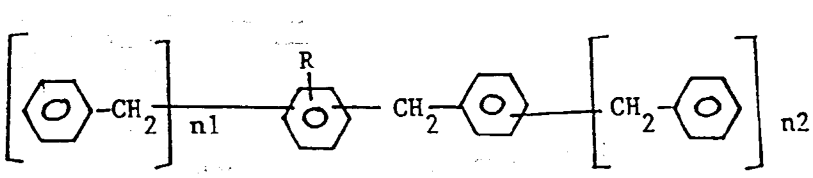

avec $n_1$ et $n_2$ = 0, 1 et 2 sachant que $n_1 + n_2$ est inférieur ou égal à 3 et R représente un hydrogène.

- $A_2$ est un isomère ou un mélange d'isomères de même formule générale que $A_1$ sauf que R représente un méthyle et $n_1$ et $n_2$ sont remplacés par $q_1$ et $q_2$ et ont la même signification,,

caractérisé en ce que :

a) on fait réagir le chlore sur du toluène en présence d'un générateur de radicaux libres,
b) on élimine le chlorure de benzylidène,
c) on ajoute du benzène et on soumet le produit obtenu à l'action d'un halogénure minéral ou d'un acide minéral.

**12.** Procédé de préparation du mélange des oligomères $A_1$, $A_2$, $B_1$, $B_2$ et $B_3$ tel que

- $A_1$ est un isomère ou un mélange d'isomères de formule :

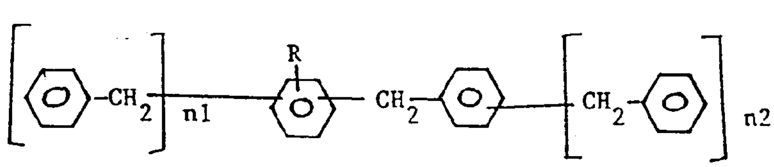

avec $n_1$ et $n_2$ = 0, 1 et 2 sachant que $n_1 + n_2$ est inférieur ou égal à 3 et R représente un hydrogène.

- $A_2$ est un isomère ou un mélange d'isomères de même formule générale que $A_1$ sauf que R représente un méthyle et $n_1$ et $n_2$ sont remplacés par $q_1$ et $q_2$ et ont la même signification,
- $B_1$ est un isomère ou un mélange d'isomères de formule :

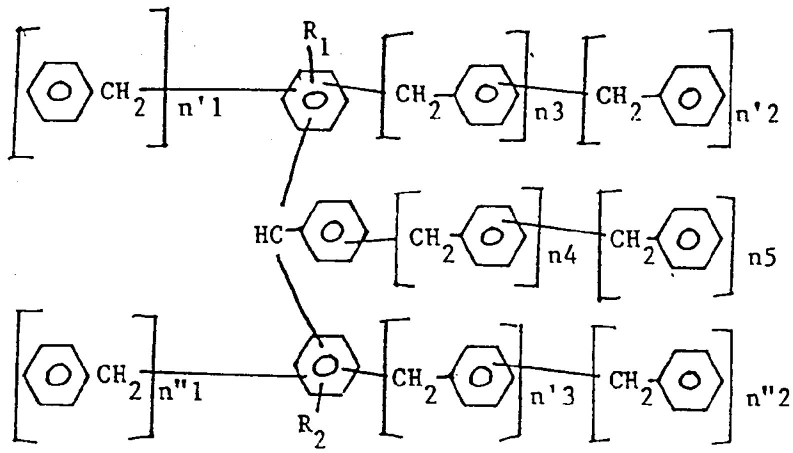

avec :

$n'_1$, $n''_1$ et $n_4$ = 0, 1 et 2

$n'_2$, $n''_2$, $n_3$, $n'_3$ et $n_5$ = 0 et 1

sachant que $n'_1 + n''_1 + n'_2 + n''_2 + n_3 + n'_3 + n_4 + n_5$ est inférieur ou égal à 2.

$R_1$ et $R_2$ représentent un hydrogène.

$B_2$ est un isomère ou un mélange d'isomères de même formule générale que $B_1$ sauf que $R_1$ et $R_2$ représentent un méthyle et les coefficients n sont remplacés par q et ont la même signification.

$B_3$ est un isomère ou un mélange d'isomères de même formule générale que $B_1$ sauf que $R_1$ et $R_2$ sont différents et représentent un hydrogène ou un méthyle et les coefficients n sont remplacés par r et ont la même signification, caractérisé en ce que :

a) on fait réagir le chlore sur un mélange de benzène et de toluène par réaction radicalaire en présence d'un générateur de radicaux libres,

b) puis on soumet le produit de réaction précédent à l'action d'un halogénure minéral ou d'un acide minéral.

## Patentansprüche

1. Zusammensetzung auf der Basis von zwei Oligomeren $A_1$ und $A_2$, wobei:
   - $A_1$ ein Isomeres oder eine Isomerenmischung der Formel:

   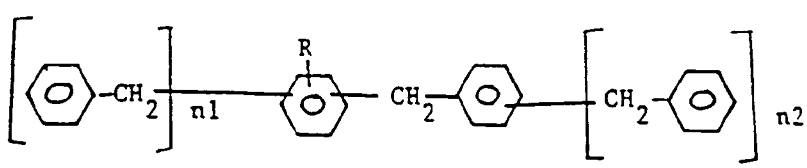

   darstellt, mit $n_1$ und $n_2$ = 0, 1 oder 2 und wobei $n_1 + n_2$ kleiner oder gleich 3 ist und R einen Wasserstoffrest darstellt.
   - $A_2$ ein Isomeres oder eine Isomerenmischung der gleichen allgemeinen Formel wie für $A_1$ darstellt, mit der Abänderung, daß R ein Methylrest ist und $n_1$ und $n_2$ durch $q_1$ und $q_2$ ersetzt werden und die gleiche Bedeutung besitzen, dadurch gekennzeichnet, daß wenigstens eines der Oligomeren $A_1$ und $A_2$ ein Isomeres mit drei Benzolringen enthält.

2. Zusammensetzung nach Anspruch 1 , dadurch gekennzeichnet, daß beim Oligomeren $A_1$ $n_1 = n_2 = 0$ und beim Oligomeren $A_2$ $q_1 + q_2 = 1$ ist.

3. Zusammensetzung nach Anspruch 1 , dadurch gekennzeichnet, daß beim Oligomeren $A_1$ $n_1 + n_2 = 1$ und beim Oligomeren $A_2$ $q_1 + q_2 = 0$ ist.

4. Zusammensetzung nach Anspruch 1 , dadurch gekennzeichnet, daß beim Oligomeren $A_1$ $n_1 + n_2 = 1$ und beim Oligomeren $A_2$ $q_1 + q_2 = 1$ ist.

5. Eine Zusammensetzung auf der Basis der zwei Oligomeren $A_1$ und $A_2$, wobei:
   - $A_1$ ein Isomeres oder eine Isomerenmischung der Formel:

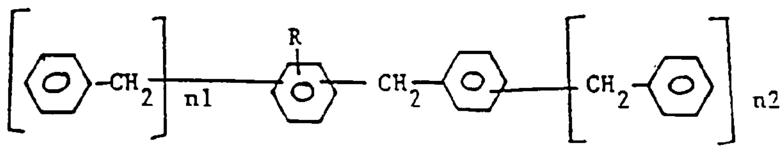

darstellt, mit $n_1$ und $n_2$= 0, 1 oder 2 und wobei $n_1 + n_2$ kleiner oder gleich 3 ist sowie R einen Wasserstoffrest darstellt.
   - $A_2$ ein Isomeres oder eine Isomerenmischung der allgemeinen Formel wie für $A_1$ darstellt, mit der Abänderung, daß R einen Methylrest darstellt und $n_1$ und $n_2$ durch $q_1$ und $q_2$ ersetzt werden und die gleiche Bedeutung besitzen, dadurch gekennzeichnet, daß sie zusätzlich mindestens ein Oligomeres, ausgewählt aus den nachstehenden Oligomeren $B_1$ , $B_2$ und $B_3$, enthält:
   - $B_1$ ist ein Isomeres oder eine Isomerenmischung der Formel:

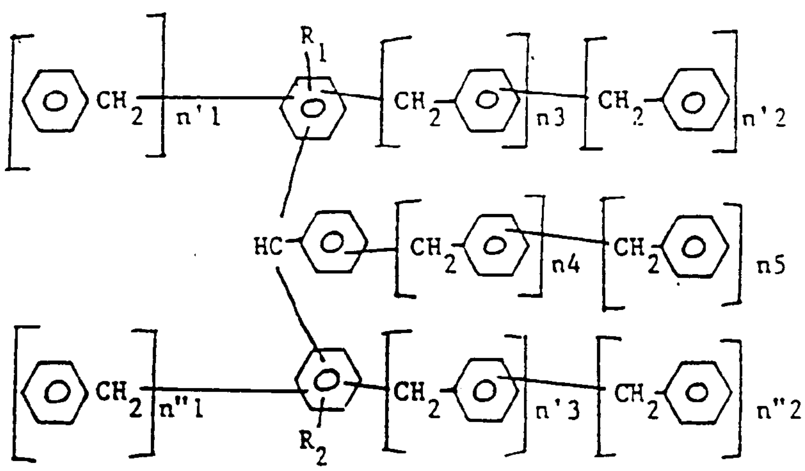

mit:
$n'_1$, $n''_1$ und $n_4$ = 0, 1 und 2,
$n'_2$, $n''_2$, $n_3$, $n'_3$ und $n_5$ = 0 und 1,
wobei $n'_1 + n''_1 + n'_2 + n''_2 + n_3 + n'_3 + n_4 + n_5$ gleich oder kleiner 2 ist.
$R_1$ und $R_2$ stellen einen Wasserstoffrest dar.
$B_2$ ist ein Isomeres oder eine Isomerenmischung der gleichen allgemeinen Formel wie für $B_1$ , mit der Abänderung, daß $R_1$ und $R_2$ einen Methylrest darstellen und die Koeffizienten n durch q ersetzt werden und die gleiche Bedeutung haben.
$B_3$ ist ein Isomeres oder eine Isomerenmischung der gleichen allgemeinen Formel wie für $B_1$, mit der Abänderung, daß $R_1$ und $R_2$ unterschiedlich sind und einen Wasserstoff- oder Methylrest darstellen und die Koeffizienten n durch r ersetzt werden und die gleiche Bedeutung besitzen.

6. Zusammensetzung nach Anspruch 5 , dadurch gekennzeichnet, daß sie die Oligomeren $A_1$, $A_2$, und $B_2$ enthält.

7. Zusammensetzung nach Anspruch 5, dadurch gekennzeichnet, daß sie die Oligomeren $A_1$, $A_2$, $B_1$, $B_2$, und

$B_3$ enthält.

8. Anwendung der Zusammensetzungen nach einem der Ansprüche 1 bis 7 als Isolierflüssigkeit (Dielektrikum).

9. Verfahren zur Herstellung der Oligomerenmischung $A_1 + A_2$, wobei:
   - $A_1$ ein Isomeres oder ein Isomerengemisch der Formel:

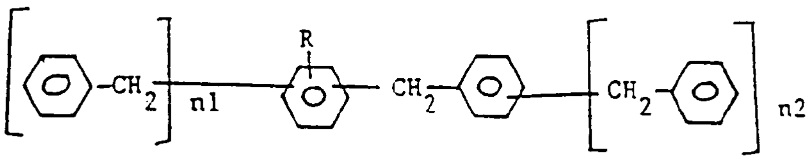

darstellt, mit $n_1$ und $n_2$ = 0, 1, oder 2 und wobei $n_1 + n_2$ kleiner oder gleich 3 ist und R einen Wasserstoffrest darstellt.
   - $A_2$ ein Isomeres oder ein Isomerengemisch der gleichen allgemeinen Formel wie für $A_1$ darstellt, mit der Abänderung, daß R einen Methylrest darstellt und $n_1$ und $n_2$ durch $q_1$ und $q_2$ ersetzt werden und die gleiche Bedeutung besitzen,

dadurch gekennzeichnet, daß man Benzylchlorid mit Benzol und Toluol in Gegenwart eines mineralischen Halogenids oder einer Mineralsäure reagieren läßt.

10. Verfahren zur Herstellung einer Mischung $A_1 + A_2$, wobei:
   - $A_1$ ein Isomeres oder eine Isomerenmischung der Formel:

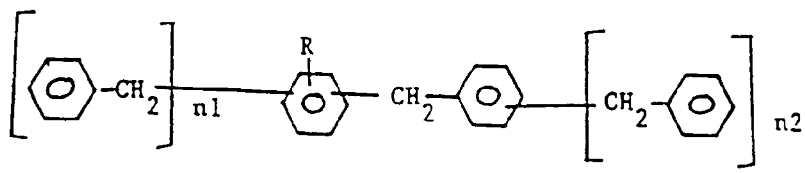

darstellt, mit $n_1$ und $n_2$ = 0, 1 und 2, wobei $n_1 + n_2$ kleiner oder gleich 3 ist und R einen Wasserstoffrest darstellt.
   - $A_2$ ein Isomeres oder ein Isomerengemisch der gleichen allgemeinen Formel wie für $A_1$ darstellt, mit der Abänderung, daß R einen Methylrest darstellt und $n_1$ und $n_2$ durch $q_1$ und $q_2$ ersetzt werden und die gleiche Bedeutung besitzen,

dadurch gekennzeichnet, daß man:

a) Chlor mit Toluol und Benzol in Gegenwart eines Radikalbildners reagieren läßt,
b) das Benzylidenchlorid entfernt,
c) das erhaltene Produkt mit einem mineralischen Halogenid oder einer Mineralsäure reagieren läßt.

11. Verfahren zur Herstellung einer Mischung $A_1 + A_2$, wobei:
   - $A_1$ ein Isomeres oder eine Isomerenmischung der Formel:

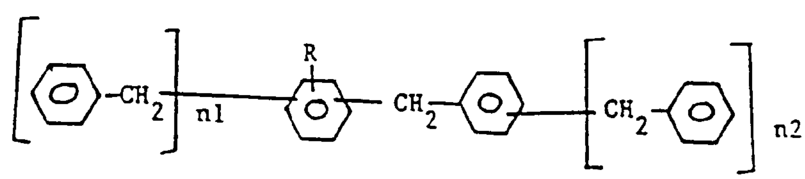

darstellt, mit $n_1$ und $n_2$ = 0, 1 und 2, wobei $n_1 + n_2$ kleiner oder gleich 3 ist und R einen Wasserstoffrest darstellt.
   - $A_2$ ein Isomeres oder ein Isomerengemisch der gleichen allgemeinen Formel wie für $A_1$ darstellt, mit

der Abänderung, daß R einen Methylrest darstellt und $n_1$ und $n_2$ durch $q_1$ und $q_2$ ersetzt werden und die gleiche Bedeutung besitzen,

dadurch gekennzeichnet, daß man

a) Chlor mit Toluol in Gegenwart eines Radikalbildners reagieren läßt,

b) das Benzylidenchlorid entfernt,

c) Benzol hinzufügt und das erhaltene Produkt mit einem mineralischen Halogenid oder einer Mineralsäure reagieren läßt.

**12.** Verfahren zur Herstellung einer Mischung der Oligomeren $A_1$, $A_2$, $B_1$, $B_2$ und $B_3$, wobei:

- $A_1$ ein Isomeres oder ein Isomerengemisch der Formel:

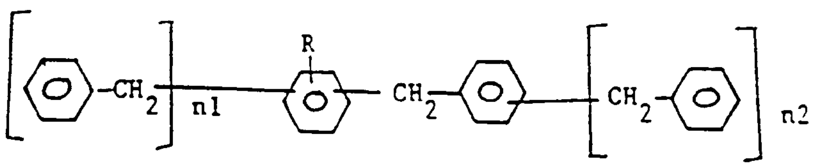

darstellt, mit $n_1$ und $n_2$ = 0, 1 und 2 und wobei $n_1 + n_2$ kleiner oder gleich 3 ist und R einen Wasserstoffrest darstellt.

- $A_2$ ein Isomeres oder ein Isomerengemisch der gleichen allgemeinen Formel wie für $A_1$ darstellt, mit der Abänderung, daß R einen Methylrest darstellt und $n_1$ und $n_2$ durch $q_1$ und $q_2$ ersetzt werden und die gleiche Bedeutung besitzen,
- $B_1$ ein Isomeres oder eine Isomerenmischung der Formel:

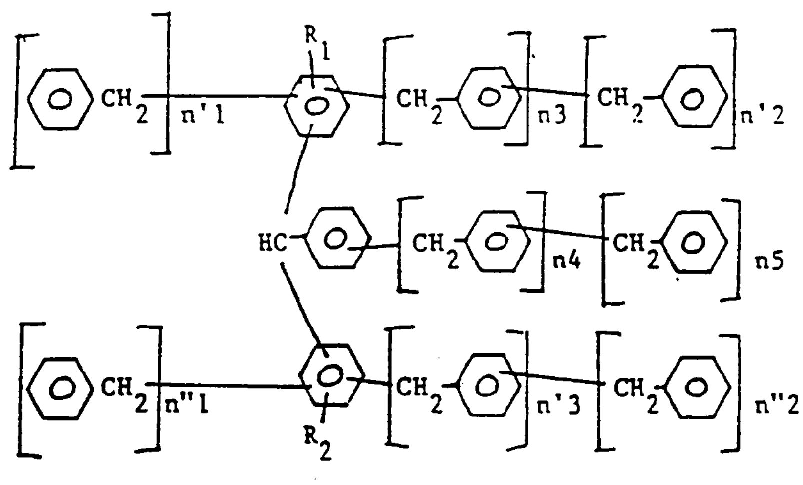

darstellt, mit:

$n'_1$, $n''_1$ und $n_4$ = 0, 1 und 2,

$n'_2$, $n''_2$, $n_3$, $n'_3$ und $n_5$ = 0 und 1,

wobei $n'_1 + n''_1 + n'_2 + n''_2 + n_3 + n'_3 + n_4 + n_5$ kleiner oder gleich 2 sind.

$R_1$ und $R_2$ stellen einen Wasserstoffrest dar.

$B_2$ ist ein Isomeres oder ein Isomerengemisch der gleichen allgemeinen Formel wie für $B_1$, mit der Abänderung, daß $R_1$ und $R_2$ einen Methylrest darstellen und die Koeffizienten n durch q ersetzt werden und die gleiche Bedeutung besitzen.

$B_3$ ist ein Isomeres oder eine Isomerenmischung der gleichen allgemeinen Formel wie für $B_1$, mit der Abänderung, daß $R_1$ und $R_2$ unterschiedlich sind und einen Wasserstoff- oder Methylrest darstellen sowie daß die Koeffizienten n durch r ersetzt werden und die gleiche Bedeutung besitzen,

dadurch gekennzeichnet, daß man :

a) Chlor mit einer Mischung aus Benzol und Toluol durch radikalische Reaktion in Gegenwart eines Radikalbildners reagieren läßt,

b) anschließend das durch die vorstehende Reaktion erhaltene Produkt mit einem mineralischen Halogenid oder einer Mineralsäure reagieren läßt.

## Claims

1. Composition based on two oligomers $A_1$ and $A_2$ such that:
   - $A_1$ is an isomer or a mixture of isomers of formula:

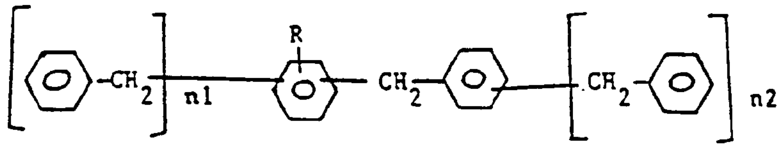

   with $n_1$ and $n_2$ = 0, 1 and 2, in the knowledge that $n_1 + n_2$ is smaller than or equal to 3 and R denotes a hydrogen;
   - $A_2$ is an isomer or a mixture of isomers of the same general formula as $A_1$ except that R denotes a methyl and $n_1$ and $n_2$ are replaced by $q_1$ and $q_2$ and have the same meaning, characterised in that at least one of the oligomers $A_1$ and $A_2$ comprises an isomer which has three benzene rings.

2. Composition according to Claim 1, characterised in that the oligomer $A_1$ is such that $n_1 = n_2 = 0$ and the oligomer $A_2$ is such that $q_1 + q_2 = 1$.

3. Composition according to Claim 1, characterised in that the oligomer $A_1$ is such that $n_1 + n_2 = 1$ and the oligomer $A_2$ is such that $q_1 = q_2 = 0$.

4. Composition according to Claim 1, characterised in that the oligomer $A_1$ is such that $n_1 + n_2 = 1$ and the oligomer $A_2$ is such that $q_1 + q_2 = 1$.

5. A composition based on the two oligomers $A_1$ and $A_2$ such that
   - $A_1$ is an isomer or a mixture of isomers of formula:

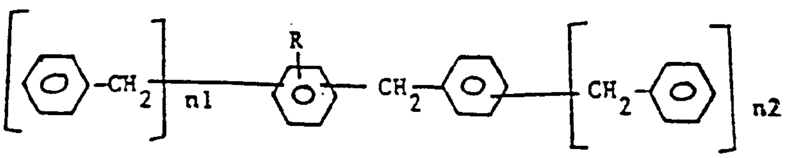

   with $n_1$ and $n_2$ = 0, 1 and 2, in the knowledge that $n_1 + n_2$ is smaller than or equal to 3 and R denotes a hydrogen;
   - $A_2$ is an isomer or a mixture of isomers of the same general formula as $A_1$ except that R denotes a methyl and $n_1$ and $n_2$ are replaced by $q_1$ and $q_2$ and have the same meaning, characterised in that it additionally comprises at least one oligomer chosen from the following oligomers $B_1$, $B_2$ and $B_3$:
   - $B_1$ is an isomer or a mixture of isomers of formula:

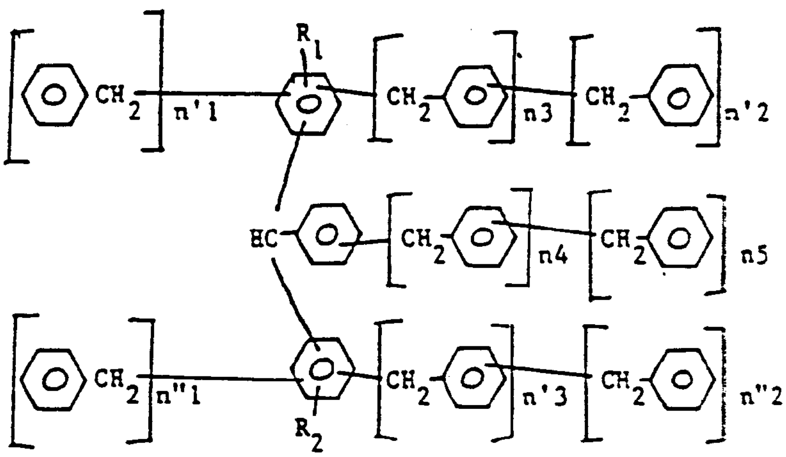

with:

$n'_1$, $n''_1$ and $n_4$ = 0, 1 and 2

$n'_2$, $n''_2$, $n_3$, $n'_3$ and $n_5$ = 0 and 1 in the knowledge that $n'_1 + n''_1 + n'_2 + n''_2 + n_3 + n'_3 + n_4 + n_5$ is smaller than or equal to 2;

$R_1$ and $R_2$ denote a hydrogen;

$B_2$ is an isomer or a mixture of isomers of the same general formula as $B_1$ except that $R_1$ and $R_2$ denote a methyl and the coefficients n are replaced by q and have the same meaning;

$B_3$ is an isomer or a mixture of isomers of the same general formula as $B_1$ except that $R_1$ and $R_2$ are different and denote a hydrogen or a methyl and the coefficients n are replaced by r and have the same meaning.

6. Composition according to Claim 5, characterised in that it comprises the oligomers $A_1$, $A_2$ and $B_2$.

7. Composition according to Claim 5, characterised in that it comprises the oligomers $A_1$, $A_2$, $B_1$, $B_2$ and $B_3$.

8. Application of the compositions according to one of Claims 1 to 7 as a dielectric fluid.

9. Process for preparing the mixture of oligomers $A_1 + A_2$ such that
   - $A_1$ is an isomer or a mixture of isomers of formula:

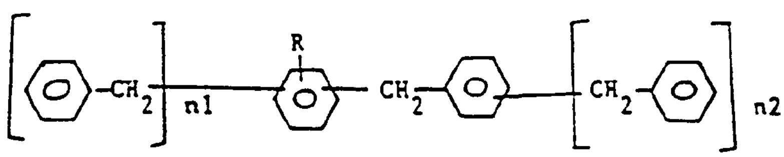

with $n_1$ and $n_2$ = 0, 1 and 2, in the knowledge that $n_1 + n_2$ is smaller than or equal to 3 and R denotes a hydrogen;

   - $A_2$ is an isomer or a mixture of isomers of the same general formula as $A_1$ except that R denotes a methyl and $n_1$ and $n_2$ are replaced by $q_1$ and $q_2$ and have the same meaning, characterised in that benzyl chloride is brought into contact with benzene and toluene in the presence of an inorganic halide or of an inorganic acid.

10. Process for preparing a mixture $A_1 + A_2$, such that
   - $A_1$ is an isomer or a mixture of isomers of formula:

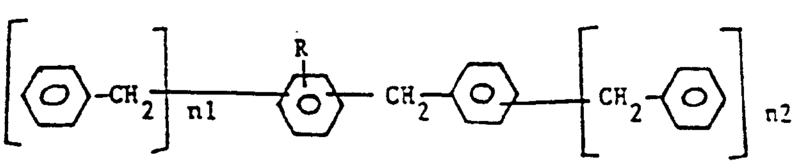

with $n_1$ and $n_2$ = 0, 1 and 2, in the knowledge that $n_1 + n_2$ is smaller than or equal to 3 and R denotes a hydrogen;

- $A_2$ is an isomer or a mixture of isomers of the same general formula as $A_1$ except that R denotes a methyl and $n_1$ and $n_2$ are replaced by $q_1$ and $q_2$ and have the same meaning, characterised in that:
  a) chlorine is reacted with toluene and benzene in the presence of a free radical generator,
  b) benzylidene chloride is removed,
  c) the product obtained is subjected to the action of an inorganic halide or of an inorganic acid.

**11.** Process for preparing a mixture $A_1 + A_2$ such that
- $A_1$ is an isomer or a mixture of isomers of formula:

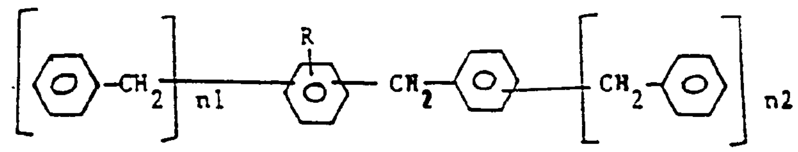

with $n_1$ and $n_2$ = 0, 1 and 2, in the knowledge that $n_1 + n_2$ is smaller than or equal to 3 and R denotes a hydrogen;

- $A_2$ is an isomer or a mixture of isomers of the same general formula as $A_1$ except that R denotes a methyl and $n_1$ and $n_2$ are replaced by $q_1$ and $q_2$ and have the same meaning, characterised in that:
  a) chlorine is reacted with toluene in the presence of a free radical generator,
  b) benzylidene chloride is removed,
  c) benzene is added and the product obtained is subjected to the action of an inorganic halide or of an inorganic acid.

**12.** Process for preparing the mixture of the oligomers $A_1$, $A_2$, $B_1$, $B_2$ and $B_3$ such that
- $A_1$ is an isomer or a mixture of isomers of formula:

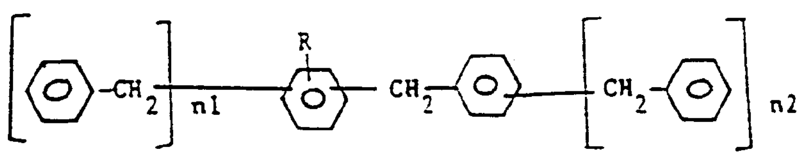

with $n_1$ and $n_2$ = 0, 1 and 2, in the knowledge that $n_1 + n_2$ is smaller than or equal to 3 and R denotes a hydrogen;

- $A_2$ is an isomer or a mixture of isomers of the same general formula as $A_1$ except that R denotes a methyl and $n_1$ and $n_2$ are replaced by $q_1$ and $q_2$ and have the same meaning,
- $B_1$ is an isomer or a mixture of isomers of formula:

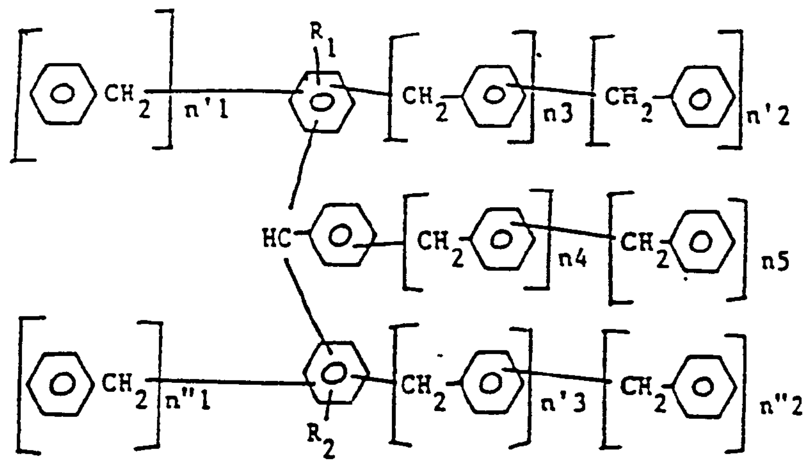

with:

$n'_1$, $n''_1$ and $n_4$ = 0, 1 and 2

$n'_2$, $n''_2$, $n_3$, $n'_3$ and $n_5$ = 0 and 1

in the knowledge that $n'_1 + n''_1 + n'_2 + n''_2 + n_3 + n'_3 + n_4 + n_5$ is smaller than or equal to 2;

$R_1$ and $R_2$ denote a hydrogen;

$B_2$ is an isomer or a mixture of isomers of the same general formula as $B_1$ except that $R_1$ and $R_2$ denote a methyl and the coefficients n are replaced by q and have the same meaning;

$B_3$ is an isomer or a mixture of isomers of the same general formula as $B_1$ except that $R_1$ and $R_2$ are different and denote a hydrogen or a methyl and the coefficients n are replaced by r and have the same meaning, characterised in that:

a) chlorine is reacted with a mixture of benzene and toluene by a radical reaction in the presence of a free radical generator,

b) the above reaction product is then subjected to the action of an inorganic halide or of an inorganic acid.